# EUROPEAN PATENT APPLICATION

(11) **EP 4 850 610 A1**
(43) Date of publication of application: **09.12.2026**
(21) Application number: 25180227.8
(22) Date of filing: 02.06.2025
(51) Int. Cl.: A61B 8/04, A61B 5/00, A61B 8/08, A61B 8/00, G06N 20/10

(54) **NON-INVASIVE PREDICTION OF LEFT VENTRICULAR END DIASTOLIC PRESSURE THROUGH MULTI-PARAMETER ULTRASOUND MEASUREMENTS**

(71) Applicant: Koninklijke Philips N.V., 5656 AE Eindhoven (NL)
(72) Inventor: SU, Jimmy Li-Shin, Eindhoven (NL); PRATER, David, Eindhoven (NL); PRABHU, David, 5656AG Eindhoven (NL)
(74) Representative: Philips Intellectual Property & Standards

(57) **Abstract**

An ultrasound imaging system may receive ultrasound data of a heart, wherein the ultrasound data was acquired across at least a portion of a cardiac cycle of the heart. The ultrasound imaging system may generate a plurality of heart based measurements based, at least in part on the ultrasound data. The ultrasound imaging system may analyze, using a correlation algorithm, the plurality of heart based measurements to determine a value of cardiac pressure. The correlation algorithm is a support vector machine (SVM) model. The plurality of heart based measurements comprise at least two of a left atrial (LA) index, a left ventricular (LV) index, LA volume parameters, echo parameters, LV strain data, LA strain data, right atrial (RA) strain data, right ventricular (RV) strain data, ultrasound Doppler data, ultrasound pulsed wave Doppler data, ultrasound continuous wave Doppler data, ultrasound two dimensional color data, and ultrasound three dimensional color data. In some cases, the ultrasound imaging system may weight one or more of the plurality of heart based measurements.

## Description

### FIELD OF THE INVENTION

The present disclosure pertains to imaging systems and methods for non-invasively measuring pressure. More specifically, the present disclosure pertains to analyzing ultrasound data to non-invasively measure left ventricular pressure.

### BACKGROUND OF THE INVENTION

Left ventricular end diastolic pressure (LVEDP) is an important clinical measure to determine ventricular function, and this measure can help physicians determine patients who are at risk for late stage cardiac failure. LVEDP is traditionally obtained through left heart catheterization, which is an invasive procedure and requires additional time and resources that could be spent treating the patient. It would be desirable to obtain LVEDP through non-invasive methods. Several methods have been previously proposed based on simple regression techniques that involve manual selection of a small number of input parameters and then somewhat optimized amongst these parameters. However, these methods are not ideal for such a multi-dimensional problem that is affected by many different aspects over the cardiac cycle, especially when two or more of the input parameters may not be independent.

### SUMMARY OF THE INVENTION

An ultrasound imaging system may receive ultrasound data of a heart, wherein the ultrasound data was acquired across at least a portion of a cardiac cycle of the heart. The ultrasound imaging system may generate a plurality of heart based measurements based, at least in part on the ultrasound data. The ultrasound imaging system may analyze, using a correlation algorithm, the plurality of heart based measurements to determine a value of cardiac pressure. In some examples, the correlation algorithm is a support vector machine (SVM) model trained on one or more of a left atrial (LA) index, a left ventricular (LV) index, LA volume parameters, echo parameters, LV strain data, LA strain data, right atrial (RA) strain data, and right ventricular (RV) strain data. The LA index and/or the LV index may be understood as a quantitative measure that is derived/trained from other echo parameters pertaining to the LA or LV, respectively. In some cases, the ultrasound imaging system may weight one or more of the plurality of heart based measurements.

The invention is defined by the independent claims. Advantageous embodiments are provided in the dependent claims.

In accordance with an example disclosed herein, an ultrasound imaging system is disclosed. The ultrasound imaging system comprises a processor configured to receive ultrasound data of a heart, wherein the ultrasound data was acquired across at least a portion of a cardiac cycle of the heart. The processor is further configured to generate a plurality of heart based measurements based, at least in part on the ultrasound data. The processor is further configured to analyze, using a correlation algorithm, the plurality of heart based measurements to determine a value of cardiac pressure. In particular, the correlation algorithm comprises a support vector machine (SVM) model. Moreover, the plurality of heart based measurements comprise at least two of a left atrial (LA) index, a left ventricular (LV) index, LA volume parameters, echo parameters, LV strain data, LA strain data, right atrial (RA) strain data, right ventricular (RV) strain data, ultrasound Doppler data, ultrasound pulsed wave Doppler data, ultrasound continuous wave Doppler data, ultrasound two dimensional color data, and ultrasound three dimensional color data.

In some embodiments, the support vector machine (SVM) model is trained on one or more of a left atrial (LA) index, a left ventricular (LV) index, LA volume parameters, echo parameters, LV strain data, LA strain data, right atrial (RA) strain data, right ventricular (RV) strain data, ultrasound Doppler data, ultrasound pulsed wave Doppler data, ultrasound continuous wave Doppler data, ultrasound two dimensional color data, and ultrasound three dimensional color data.

In some embodiments, the processor is further configured to weight one or more of the plurality of heart based measurements and analyze, using the correlation algorithm, the weighted one or more of the plurality of heart based measurements to determine the value of cardiac pressure.

In some embodiments, the plurality of heart based measurements comprises one or more global left ventricular (LV) strain curves, and wherein the one or more global LV strain curves comprise an average of a plurality of LV strain curves.

In some embodiments, the correlation algorithm may further comprise at least one of a partial least squares model or a long short-term memory network.

In some embodiments, the processor is further configured to analyze a sequence of two-dimensional ultrasound images with a machine learning model to determine a border of a chamber of the heart in individual ones of the two-dimensional ultrasound images and calculate volumes of the chamber, based, at least in part, on the borders of the individual ones of the two-dimensional ultrasound images, wherein the volumes of the chamber are included in the ultrasound data.

In some embodiments, the processor is further configured to analyze a sequence of three-dimensional ultrasound images with a machine learning model to determine a border of a chamber of the heart in individual ones of the three-dimensional ultrasound images and calculate volumes of the chamber, based, at least in part, on the borders of the individual ones of the three-dimensional ultrasound images, wherein the volumes of the chamber are included in the ultrasound data.

In accordance with another example disclosed herein, a method is disclosed. The method comprises receiving ultrasound data of a heart, wherein the ultrasound data was acquired across at least a portion of a cardiac cycle of the heart. The method further comprises generating a plurality of heart based measurements based, at least in part on the ultrasound data. The method further comprises analyzing, using a correlation algorithm, the plurality of heart based measurements to determine a value of cardiac pressure. In particular, the correlation algorithm comprises a support vector machine (SVM) model. Moreover, the plurality of heart based measurements comprise at least two of a left atrial (LA) index, a left ventricular (LV) index, LA volume parameters, echo parameters, LV strain data, LA strain data, right atrial (RA) strain data, and right ventricular (RV) strain data. , ultrasound Doppler data, ultrasound pulsed wave Doppler data, ultrasound continuous wave Doppler data, ultrasound two dimensional color data, and ultrasound three dimensional color data.

In some embodiments, the support vector machine (SVM) model is trained on one or more of a left atrial (LA) index, a left ventricular (LV) index, LA volume parameters, echo parameters, LV strain data, LA strain data, right atrial (RA) strain data, right ventricular (RV) strain data, ultrasound Doppler data, ultrasound pulsed wave Doppler data, ultrasound continuous wave Doppler data, ultrasound two dimensional color data, and ultrasound three dimensional color data.

In some embodiments, the method further comprises: weighting one or more of the plurality of heart based measurements; and analyzing, using the correlation algorithm, the weighted one or more of the plurality of heart based measurements to determine the value of cardiac pressure.

In some embodiments, the plurality of heart based measurements comprises one or more global LV strain curves, and wherein the one or more global LV strain curves comprise an average of a plurality of LV strain curves.

In some embodiments, the method further comprises filtering the ultrasound data with a digital filter, and wherein the digital filter includes a Savitsky-Golay filter with a cubic polyfit.

In some embodiments, the method further comprises interpolating the ultrasound data to a pre-set number of frames across the at least the portion of the cardiac cycle.

In some embodiments, the method further comprises generating a classifier associated with the value of the cardiac pressure.

In some embodiments, the method further comprises: analyzing a sequence of ultrasound images with a machine learning model to determine a border of a chamber of the heart in individual ones of the ultrasound images; and calculating volumes of the chamber, based, at least in part, on the borders of the individual ones of the ultrasound images, wherein the volumes of the chamber are included in the ultrasound data. In some such embodiments, the sequence of ultrasound images may comprise three-dimensional ultrasound images and/or two-dimensional ultrasound images.

In accordance with yet another example disclosed herein, a non-transitory computer-readable medium is disclosed. The non-transitory computer-readable medium carries instructions which, when executed by a processor, cause the processor to carry out the steps of any of the methods described above.

In some embodiments, the processor is a processor of an ultrasound imaging system, such as any of the systems described above. In such embodiments, the instructions, when executed by the processor, cause the ultrasound imaging system to carry out the steps of any of the methods described above.

### BRIEF DESCRIPTION OF THE DRAWINGS

To easily identify the discussion of any particular element or act, the most significant digit or digits in a reference number refer to the figure number in which that element is first introduced.
Fig. 1 is a block diagram of an ultrasound system in accordance with principles of the present disclosure.
Fig. 2 is a block diagram illustrating an example processor in accordance with principles of the present disclosure.
   Fig. 3 is a block diagram of a process for training and deployment of a neural network in accordance with the principles of the present disclosure.
Fig. 4 is an example of a strain curve in accordance with the principles of the present disclosure.
Fig. 5 illustrates an example of a longitudinal global strain curve in accordance with the principles of the present disclosure.
Fig. 6 is an illustration of a neural network that may be used to data in accordance with examples of the present disclosure.
Fig. 7 is an illustration of a cell of a long short term memory model that may be used to analyze data in accordance with examples of the present disclosure.
Fig. 8 illustrates an example SVM model in accordance with the principles of the present disclosure.
Fig. 9 illustrates a method, according to embodiments herein.

### DETAILED DESCRIPTION OF EMBODIMENTS

The following description of certain embodiments is merely exemplary in nature and is in no way intended to limit the invention or its applications or uses. In the following detailed description of embodiments of the present systems and methods, reference is made to the accompanying drawings which form a part hereof, and which are shown by way of illustration specific embodiments in which the described systems and methods may be practiced. These embodiments are described in sufficient detail to enable those skilled in the art to practice presently disclosed systems and methods, and it is to be understood that other embodiments may be utilized and that structural and logical changes may be made without departing from the spirit and scope of the present system. Moreover, for the purpose of clarity, detailed descriptions of certain features will not be discussed when they would be apparent to those with skill in the art so as not to obscure the description of the present system. The following detailed description is therefore not to be taken in a limiting sense, and the scope of the present system is defined only by the appended claims.

As described by Kawasaki et al., the left pulmonary capillary wedge pressure (PCWP) value is correlated to several aspects of the left atrium (LA) volume cycle. Kawasaki ran multiple iterations of a regression test to see which LA volume parameters would correlate the best with the PCWP catheter pressure value. In the end, Kawasaki chose one combination of two parameters to create what is known in the paper as the "KT index". *See* M. Kawasaki et al. "A novel ultrasound predictor of pulmonary capillary wedge pressure assessed by the combination of left atrial volume and function: A speckle tracking echocardiography study," J. Cardiol., vol 66. No. 3, pp. 253-262, 2015. This method, while manually optimized, ignores all the other parameters that also showed correlation to catheter pressure. To include these would have created a multi-dimensional regression equation. However, multi-dimensional linear regression cannot be used if the independent, input parameters are linearly correlated with one another, which the LA volume parameters are found to be, in this case. Furthermore, the input parameters used by Kawasaki are fraught with noise, and are not cleaned up prior to being used in his regression equation.

Additionally, it has been shown that left ventricular end diastolic pressure (LVEDP) is directly related to left atrial strain information. Previous publications have shown correlation by manual linear regression methods alone (*See e.g.,* M. Cameli et al., "Left atrial longitudinal strain by speckle tracking echocardiography correlates well with left ventricular filling pressures in patients with heart failure," Cardiovascular Ultrasound, vol 8. No 14. 2010, S.F. Nagueh et al., "Doppler Tissue Imaging: A Noninvasive Technique for Evaluation of Left Ventricular Relaxation and Estimation of Filling Pressures", JACC Vol. 30, No 6 November 15, 1997: 1527-33, and A. Singh et al., "Peak left atrial strain as a single measure for the non-invasive assessment of left ventricular filling pressures," Int J Cardiovasc Imaging, vol 35. No 1. 2019. These additional methods that develop a relationship between ventricular pressure and strain operate similarly to the LA volume methods described by Kawasaki.

A non-invasive solution that can take advantage of the information provided by multiple parameters, regardless of whether or not the parameters are independent, across multiple phases of the cardiac cycle (e.g., from early diastole to late systole, from early diastole of a first heartbeat to early diastole of a second heartbeat, etc.) is desired.

According to principles of the present disclosure, ultrasound data acquired from a heart, or one or more portions of the heart such as from the LA or left ventricle (LV), may be provided to a correlation algorithm, which may include a model (e.g., machine learning, deep learning, artificial intelligence, one or more algorithms). The model may determine a cardiac pressure (e.g., LVEDP, LV filling pressure, PCWP, Pre-A Pressure) based on the provided ultrasound data. Examples of ultrasound-derived data from the heart that may be provided to the model include, but are not limited to, tissue strain measurements (e.g., longitudinal strain, circumferential strain) and volume. In some examples, the model may include a partial least squares (PLS) model. In some examples, the model may be trained to generate a transfer function which may be used to determine the pressure based on the provided ultrasound data. In some examples, the transfer function may include one or more regression coefficients. In some examples, the model may include a neural network, such as a long short-term memory (LSTM) network. In some examples, the model may output a numeric value for the pressure. In some examples, the model may output a classifier that provides a qualitative indication of the pressure (e.g., normal, high, low).

In some examples, the ultrasound data may be preprocessed prior to being provided to the model. In some examples, the ultrasound data may be interpolated to a pre-set number of frames across a cardiac cycle. In some examples, the pre-set number of frames may be evenly spaced across the cardiac cycle. In some examples, the cardiac cycle may be sub-divided into different phases of the LA (i.e. reservoir, conduit and contraction phases), LV, and/or other portion of the heart. In some examples, these phases may each be interpolated to a pre-set number of frames individually, and then combined together. In some examples, the pre-set number of frames may be selected from desired phases of the cardiac cycle (e.g., early atrial systole, late atrial diastole). The length and/or phases of the cardiac cycle may be determined based on ultrasound imaging (e.g., B-mode or Doppler) and/or based on electrocardiography (ECG) signals (e.g., detecting the QRS wave complex). In some examples, the ultrasound data may be smoothed by a filter, such as a Savitsky-Golay filter. In some examples, the filtering may be performed after the interpolation.

In various embodiments, a multi-parametric approach may be used by the correlation algorithm to determine pressure (e.g., LVEDP). For example, the correlation algorithm may first determine a plurality of input parameters (e.g., from ultrasound data). The correlation algorithm weights each of the plurality of input parameters and uses the weighted input parameters to determine the LVEDP. It may be that, in such embodiments, the determined LVEDP value is more accurate compared to current methods for determining LVEDP as the multiple input parameters give more context and data to the correlation algorithm to produce a more accurate LVEDP value.

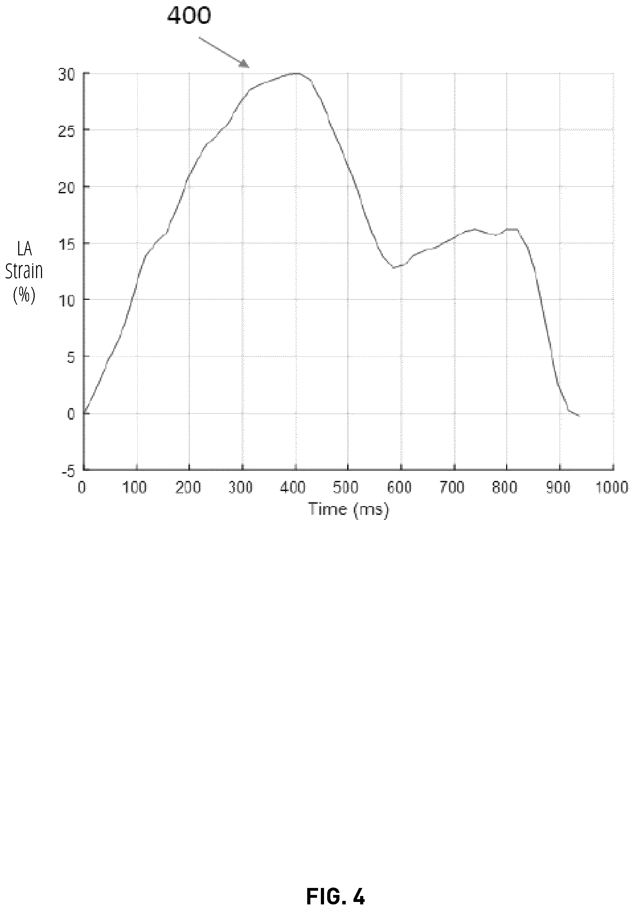

In some instances, the plurality of input parameters include various parameters that may be generated based on the ultrasound data. For example, the input parameters may include strain data such as an LA index, an LV index, LV strain data, LA strain data, RV strain data, and RA strain data (e.g., strain curves illustrated in Fig. 4) at either an end-systolic strain (ESS) or peak-systolic strain (PSS). The LA index and/or the LV index may be understood as a quantitative measure that is derived/trained from other echo parameters pertaining to the LA or LV, respectively. The input parameters may include echo parameters such as ventricular dimensions, LA volume parameter, valvular measurements, and Doppler flow parameters. Further, the input parameters may include various echo parameters. The input parameters may include ultrasound Doppler data, ultrasound pulsed wave Doppler data, ultrasound continuous wave Doppler data, ultrasound two dimensional color data, and ultrasound three dimensional color data. Additionally or alternatively, the input parameters may include previously obtained patient data such as height, weight, medical history, present diseases and/or conditions, etc. In some cases, the plurality of input parameters may be filtered (as discussed herein) to reduce noise and remove any anomalies from the input data thus providing clean input data to the multi-parametric correlation algorithm. As a result of using cleaner input data, the output determination of the multi-parametric correlation algorithm may be more accurate.

In various embodiments, the multi-parametric correlation algorithm may be used to determine various heart related or non-heart related conditions and/or diagnoses. For example, the multi-parametric correlation algorithm may be implemented to detect a cardiotoxicity level, a level of heart failure, heart disease, and/or amyloidosis. In some cases, the multi-parametric correlation algorithm may continuously detect the level of the condition and/or diagnoses.

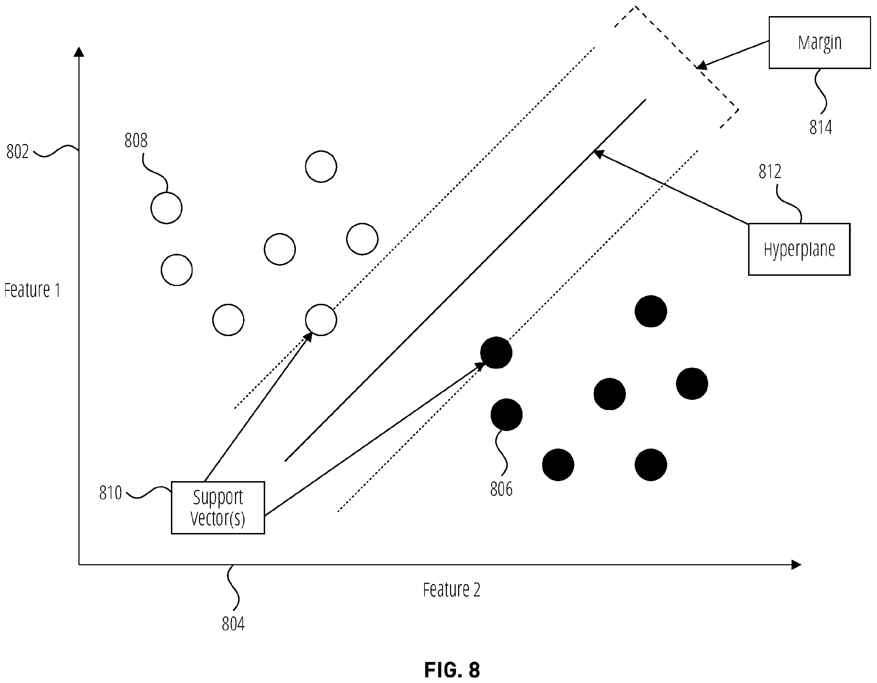

In various embodiments, the multi-parametric correlation algorithm may be understood as a support vector machine (SVM) model (e.g., illustrated in Fig. 8). The weighted input parameters are used as inputs to the SVM model and the SVM model determines/detects/predicts the condition and/or diagnoses. In some examples, the SVM model is trained based on various previously annotated ultrasound data and weighted input parameters, and ground truth data related to the condition and/or diagnosis that is to be determined/detected/predicted by the SVM model.

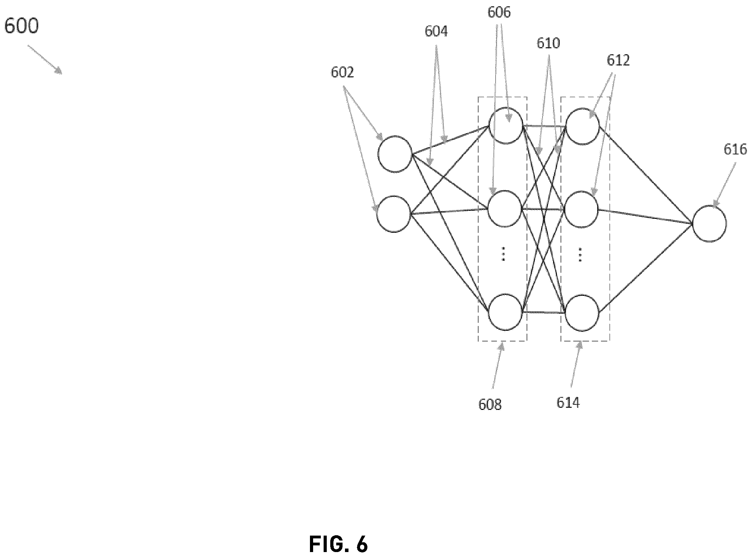

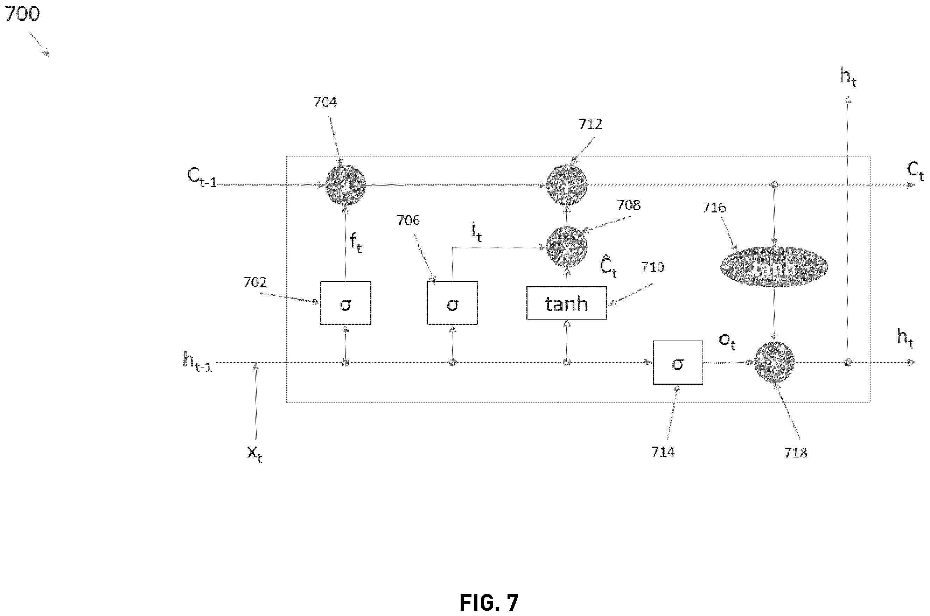

In various instances, the multi-parametric correlation algorithm may be understood as a machine learning algorithm or an artificial intelligence algorithm that can be used to find a multidimensional parameter that describes the progression of the condition and/or diagnosis (e.g., normal/high/low blood pressure, heart failure, cardiotoxicity), providing a continuous variable for the medical professional to monitor the patient over time. In various instances, the multi-parametric correlation algorithm may take the form of a neural network as illustrated in Fig. 6, a long short-term memory network as illustrated in Fig. 7, a SVM model as illustrated in Fig. 8 (as discussed), or the PLS model discussed herein.

In some instances, the multi-parametric correlation algorithm is trained with a dataset comprising manually annotated assessments of detected conditions and/or diagnoses, strain data (e.g., LV strain data, LA strain data, RV strain data, and/or RA strain data), echo parameters in previously acquired ultrasound data and/or other previously obtained input parameters discussed herein. As such, the multi-parametric correlation algorithm is trained to identify features in the ultrasound data that correlate to the manually annotated assessments of the condition and/or diagnosis that it has been trained with.

In various embodiments, the output of the multi-parametric correlation algorithm may be a classification of the condition or diagnosis. For example, for cardiac function, the outputted classification may be that the blood pressure is normal, low, or high. In another example, the classification may be that cardiac function is normal or impaired. In some instances, the output of the multi-parametric correlation algorithm may be a continuous variable (e.g., in real time if the multi-parametric correlation algorithm is implemented for real time ultrasound procedures) which can be tied to clinical outcomes, in order to provide a threshold for when treatment is required. In some instances, the output of the multi-parametric correlation algorithm may be a dimension-less value (e.g., identifier) such as a "condition detected" or "condition not detected" or the output may be a categorical prediction.

In various embodiments, the multi-parametric correlation algorithm may be incorporated for both real-time and non-real time scenarios. For example, the multi-parametric correlation algorithm can be used on a ultrasound system in real time so that when the optimal (e.g., best for strain curve generation) echo views are acquired (e.g., apical-2-chamber (AP2), apical-4-chamber (AP4), etc), the strain analysis (e.g., strain measurements and/or strain curve generation) may be computed right away. The ultrasound system implementing the multi-parametric correlation algorithm may be a premium (e.g., EPIQ^{®}), high-end (e.g., Affiniti^{®}), or point of care system (e.g., Lumify^{®}). Philips^{®}'s EPIQ^{®}, Affiniti^{®}, and Lumify^{®} systems are provided merely as examples, and other ultrasound systems may be used without departing from the principles of the present invention. In some cases, the multi-parametric correlation algorithm may be incorporated into an on-cart ultrasound system (e.g., patient side). For example, the ultrasound data for the multi-parametric correlation algorithm is obtained from echo data information acquired from diagnostic equipment (e.g. ultrasound machine) using the AutoStrain LV discussed herein. The multi-parametric correlation algorithm automatically analyzes the ultrasound data as it is being obtained and outputs the condition/diagnosis (e.g., a level of the condition/diagnosis) in some examples, in real time.

In some cases, the multi-parametric correlation algorithm may be incorporated into an off-cart workstation (e.g. TOMTEC, or Ultrasound Workspace) for offline analysis of previously acquired ultrasound data. For example, the multi-parametric correlation algorithm may analyze previously acquired ultrasound data and detect/output a condition/diagnosis level for use by a medical professional (e.g., to present at an upcoming appointment or use for other medical determinations).

In various embodiments, additional echocardiographic, physiological, or medical parameters may be used by the multi-parametric correlation algorithm (e.g., LVEF or echo doppler measurements) as additional inputs to detect the condition/diagnosis. In such embodiments, the multi-parametric correlation algorithm may take the form of an SVM model as to take into account the multiple parameters to detect the condition/diagnosis. Note that the multi-parametric correlation algorithm or the user of the algorithm may weigh the input parameters according to importance/relevance to detecting the pressure or other feature (e.g., cardiotoxicity). In some examples, the weights may be predicted by a machine learning model that is a part of the multi-parametric correlation algorithm or separate from the multi-parametric correlation algorithm.

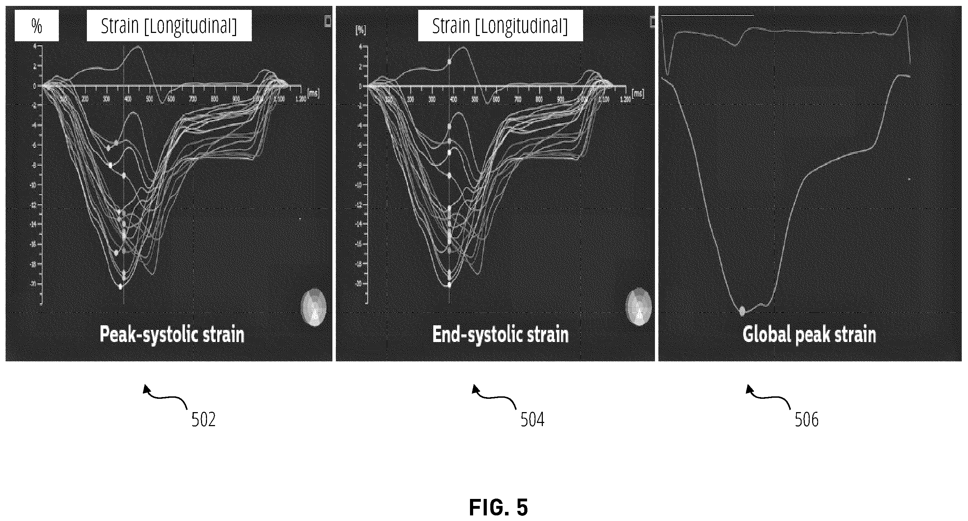

In various embodiments, a global strain curve, as illustrated in Fig. 5, may be used as an additional input to the multi-parametric correlation algorithm. The global strain curve is an average of multiple (e.g., eighteen) strain curves (e.g., LV strain curves) generated from ultrasound data. In some instances, the multiple strain curves may be used as an input to the multi-parametric correlation algorithm to either train the multi-parametric correlation algorithm or as an additional input when detecting the condition/diagnosis.

The systems and methods disclosed herein may introduce an algorithm for a multi-parametric approach for detecting/predicting/determining conditions and/or diagnoses through the use of artificial intelligence with ultrasound data over an entire phase of the cardiac cycle, multiple phases of the cardiac cycle, an entire cardiac cycle, and/or multiple cardiac cycles. In some applications, this may provide more accurate and/or consistent measurements of conditions and/or diagnosis that previously did not exist.

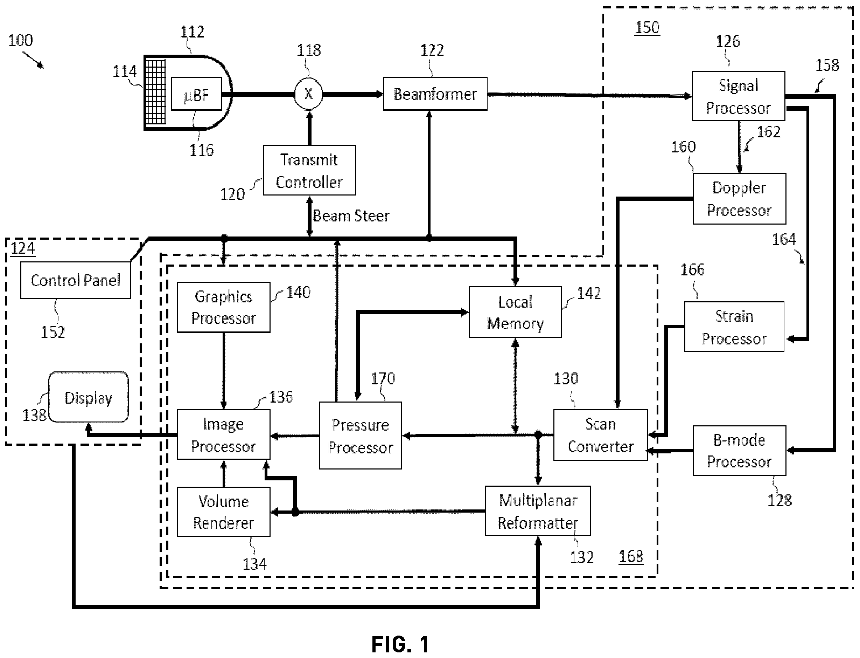

Fig. 1 shows a block diagram of an ultrasound imaging system 100 constructed in accordance with the principles of the present disclosure. An ultrasound imaging system 100 according to the present disclosure may include a transducer array 114, which may be included in an ultrasound probe 112, for example an external probe or an internal probe such as an Intra Cardiac Echography (ICE) probe or a Trans Esophagus Echography (TEE) probe. In other embodiments, the transducer array 114 may be in the form of a flexible array configured to be conformably applied to a surface of subject to be imaged (e.g., patient). The transducer array 114 is configured to transmit ultrasound signals (e.g., beams, waves) and receive echoes responsive to the ultrasound signals. A variety of transducer arrays may be used, e.g., linear arrays, curved arrays, or phased arrays. The transducer array 114, for example, can include a two dimensional array (as shown) of transducer elements capable of scanning in both elevation and azimuth dimensions for 2D and/or 3D imaging. As is generally known, the axial direction is the direction normal to the face of the array (in the case of a curved array the axial directions fan out), the azimuthal direction is defined generally by the longitudinal dimension of the array, and the elevation direction is transverse to the azimuthal direction.

In some embodiments, the transducer array 114 may be coupled to a microbeamformer 116, which may be located in the ultrasound probe 112, and which may control the transmission and reception of signals by the transducer elements in the array 114. In some embodiments, the microbeamformer 116 may control the transmission and reception of signals by active elements in the array 114 (e.g., an active subset of elements of the array that define the active aperture at any given time).

In some embodiments, the microbeamformer 116 may be coupled, e.g., by a probe cable or wirelessly, to a transmit/receive (T/R) switch 118, which switches between transmission and reception and protects the main beamformer 122 from high energy transmit signals. In some embodiments, for example in portable ultrasound systems, the T/R switch 118 and other elements in the system can be included in the ultrasound probe 112 rather than in the ultrasound system base, which may house the image processing electronics. An ultrasound system base typically includes software and hardware components including circuitry for signal processing and image data generation as well as executable instructions for providing a user interface (e.g., processing circuitry 150 and user interface 124).

The transmission of ultrasonic signals from the transducer array 114 under control of the microbeamformer 116 is directed by the transmit controller 120, which may be coupled to the T/R switch 218 and a main beamformer 122. The transmit controller 120 may control the direction in which beams are steered. Beams may be steered straight ahead from (orthogonal to) the transducer array 114, or at different angles for a wider field of view. The transmit controller 120 may also be coupled to a user interface 124 and receive input from the user's operation of a user control. The user interface 124 may include one or more input devices such as a control panel 152, which may include one or more mechanical controls (e.g., buttons, encoders, etc.), touch sensitive controls (e.g., a trackpad, a touchscreen, or the like), and/or other known input devices.

In some embodiments, the partially beamformed signals produced by the microbeamformer 116 may be coupled to a main beamformer 122 where partially beamformed signals from individual patches of transducer elements may be combined into a fully beamformed signal. In some embodiments, microbeamformer 116 is omitted, and the transducer array 114 is under the control of the main beamformer 122 which performs all beamforming of signals. In embodiments with and without the microbeamformer 116, the beamformed signals of the main beamformer 122 are coupled to processing circuitry 150, which may include one or more processors (e.g., a signal processor 126, a B-mode processor 128, a Doppler processor 160, and one or more image generation and processing components 168) configured to produce an ultrasound image from the beamformed signals (e.g., beamformed RF data).

The signal processor 126 may be configured to process the received beamformed RF data in various ways, such as bandpass filtering, decimation, I and Q component separation, and harmonic signal separation. The signal processor 126 may also perform additional signal enhancement such as speckle reduction, signal compounding, and noise elimination. The processed signals (also referred to as I and Q components or IQ signals) may be coupled to additional downstream signal processing circuits for image generation. The IQ signals may be coupled to a plurality of signal paths within the system, each of which may be associated with a specific arrangement of signal processing components suitable for generating different types of image data (e.g., B-mode image data, Doppler image data). For example, the system may include a B-mode signal path 158 which couples the signals from the signal processor 126 to a B-mode processor 128 for producing B-mode image data.

The B-mode processor can employ amplitude detection for the imaging of structures in the body. The signals produced by the B-mode processor 128 may be coupled to a scan converter 130 and/or a multiplanar reformatter 132. The scan converter 130 may be configured to arrange the echo signals from the spatial relationship in which they were received to a desired image format. For instance, the scan converter 130 may arrange the echo signal into a two dimensional (2D) sector-shaped format, or a pyramidal or otherwise shaped three dimensional (3D) format. The multiplanar reformatter 132 can convert echoes which are received from points in a common plane in a volumetric region of the body into an ultrasonic image (e.g., a B-mode image) of that plane, for example as described in U.S. Pat. No. 6,443,896 (Detmer). The scan converter 130 and multiplanar reformatter 132 may be implemented as one or more processors in some embodiments.

A volume renderer 134 may generate an image (also referred to as a projection, render, or rendering) of the 3D dataset as viewed from a given reference point, e.g., as described in U.S. Pat. No. 6,530,885 (Entrekin et al.). The volume renderer 134 may be implemented as one or more processors in some embodiments. The volume renderer 134 may generate a render, such as a positive render or a negative render, by any known or future known technique such as surface rendering and maximum intensity rendering.

In some embodiments, the system may include a Doppler signal path 162 which couples the output from the signal processor 126 to a Doppler processor 160. The Doppler processor 160 may be configured to estimate the Doppler shift and generate Doppler image data. The Doppler image data may include color data which is then overlaid with B-mode (i.e. grayscale) image data for display. The Doppler processor 160 may be configured to filter out unwanted signals (i.e., noise or clutter associated with non-moving tissue), for example using a wall filter. The Doppler processor 160 may be further configured to estimate velocity and power in accordance with known techniques. For example, the Doppler processor may include a Doppler estimator such as an auto-correlator, in which velocity (Doppler frequency) estimation is based on the argument of the lag-one autocorrelation function and Doppler power estimation is based on the magnitude of the lag-zero autocorrelation function. Motion can also be estimated by known phase-domain (for example, parametric frequency estimators such as MUSIC, ESPRIT, etc.) or time-domain (for example, cross-correlation) signal processing
techniques. Other estimators related to the temporal or spatial distributions of velocity such as estimators of acceleration or temporal and/or spatial velocity derivatives can be used instead of or in addition to velocity estimators. In some embodiments, the velocity and/or power estimates may undergo further threshold detection to further reduce noise, as well as segmentation and post-processing such as filling and smoothing. The velocity and/or power estimates may then be mapped to a desired range of display colors in accordance with a color map. The color data, also referred to as Doppler image data, may then be coupled to the scan converter 130, where the Doppler image data may be converted to the desired image format and overlaid on the B-mode image of the tissue structure to form a color Doppler or a power Doppler image. In some examples, the scan converter 130 may align the Doppler image and B-mode image

In some embodiments, the system 100 may include a strain imaging signal path 164 which couples the signals from the signal processor 126 to a strain processor 166 for producing strain measurements. The strain measurements may include elastic shear modulus, Young modulus, and/or other strain measurements. In some examples, the strain measurements may be mapped to pixel color and/or intensity values to generate maps (e.g., strain maps) that may be overlaid onto B-mode and/or Doppler images. In some examples, the scan converter 130 may align the strain measurements with the B-mode and/or Doppler images.

In some examples, the strain measurements may be obtained by shear wave elastography (SWE). In SWE, the probe 112 may transmit an ultrasound signal "push pulse" that induces a shear wave in an object (e.g., tissue). Alternatively, the shear wave in the object may be generated without acoustic radiation force but via mechanical force applied externally to the object, such as by a mechanical vibrator (not shown) that compresses the object. The probe 112 may transmit additional ultrasound signals "tracking pulses" in the object at and/or adjacent to a location where the push pulse was transmitted. Echoes responsive to the tracking pulses may be received by the probe 112. Signals based on the echoes may be analyzed by the strain processor 166 to determine various properties of the shear wave as it propagated through the locations of the tracking pulses in the object. The strain processor 166 may calculate a peak displacement, phase, velocity, and/or other features of the shear wave at one or more locations of the tracking pulses. These features of the shear wave may then be used by the strain processor 166 to calculate material properties of the object at the location of the push pulse and/or location(s) of the tracking pulses. For example, the velocity of the shear wave may be used to determine the shear modulus and/or the Young modulus. In some examples, B-mode signals and/or RF-signals may be provided to the strain processor 166 for analysis to generate strain measurements. For example, correlation between RF echo signals of different windows before and after compression may be used to determine tissue displacement. The tissue displacement may be used to calculate normal strain in some examples. In another example, speckle tracking in B-mode signals may be used to determine particle motion and/or tissue displacement. In some examples, the velocity of the particle motion may be used to calculate the bulk modulus. Other techniques of calculating strain measurements may also be used (e.g., acoustic radiation force impulse strain imaging, transient elastography).

According to embodiments of the present disclosure, output from the scan converter 130, such as B-mode images, Doppler images, strain measurements, and/or strain maps, may be provided to a pressure processor 170. The pressure processor 170 may analyze the ultrasound images, strain measurements, and/or strain maps for ultrasound data to determine a value of a heart related or non-heart related condition/diagnosis.

In some examples, the ultrasound data may include volume. In some examples, the pressure processor 170 may receive a sequence of 2D B-mode images acquired across a cardiac cycle, a portion of a cardiac cycle, and/or multiple cardiac cycles. The sequence may have been acquired at a same imaging plane. The pressure processor 170 may analyze the 2D B-mode images to find a border of the LA and/or other chambers of the heart. The border may be found using any suitable technique. For example, the border may be found using 2D Cardiac Performance Analysis provided by TOMTEC. Once the border has been determined, a volume of the chamber may be estimated, for example, using Simpson's method of disks technique. In some examples, the pressure processor 170 may receive a sequence of 3D B-mode volume images across a cardiac cycle. The pressure processor 170 may analyze the 3D B-mode volumes to find the heart chamber volume directly. The volume may be found using any suitable technique, for example, using the HeartModel^{A.I.} developed by Koninklijke Philips. Thus, the volume for the heart chamber(s) for multiple time-points across the cardiac cycle may be obtained.

In some examples, the pressure processor 170 may receive a sequence of strain maps and/or strain measurements acquired at different time points across a cardiac cycle, a portion of a cardiac cycle, and/or multiple cardiac cycles. The strain maps and/or strain measurements may be analyzed by the pressure processor 170 to generate a strain curve. The strain curve plots strain measurements over time. The strain curve may be for longitudinal or circumferential strain in some examples. An example longitudinal strain curve 400 for the LA is shown in Fig. 4. The example strain curve 400 reflects the percentage strain of the LA tissue over time in milliseconds. The strain curve may be generated by any suitable technique, for example, using AutoStrain available from TOMTEC.

The ultrasound data, such as the volume and/or strain, may be analyzed by the pressure processor 170 to determine a value for the pressure. In some examples, the ultrasound data may be preprocessed prior to being analyzed. In some applications, preprocessing the data may increase consistency in the data across data sets and/or reduce noise that may affect the determination of the pressure. In some examples, the ultrasound data may be interpolated to a pre-set number of frames across a desired acquisition length (e.g., an entire cardiac cycle, one or more phases of the cardiac cycle, multiple cardiac cycles). In some examples, interpolating may be used to increase the number of frames analyzed by the pressure processor 170 compared to the original number of frames acquired. In some examples, interpolating may ensure that a same number of data points are analyzed by the pressure processor 170 each time. In some examples, this may help improve consistency as to how the analyzed data points are distributed across the acquisition period. The pre-set number of frames may be preprogrammed in the ultrasound imaging system 100 and/or may be selected by a user via the user interface 124. In some examples, when ultrasound images are acquired over multiple cardiac cycles, the frames over the cardiac cycles may be averaged prior to interpolation. In other examples, interpolation may be performed for each cardiac cycle and the interpolated frames for the cardiac cycles may be averaged.

In some examples, the ultrasound data may be filtered. In some examples, the ultrasound data may be smoothed by a digital filter. An example of a suitable filter is a Savitsky-Golay filter. In some examples, the Savitsky-Golay filter may be used with a cubic polyfit. The window of the filter may be selected empirically in some examples. The length of the window may be based, at least in part, on the ultrasound data to be analyzed. For example, a 9-point window may be used for strain measurements in some applications and a 5-point window may be used for volume measurements in some applications. In some examples, the length of the window may be selected by a user via the user interface 124. Unlike some other smoothing filters, the Savitsky-Golay filter may reduce noise from the signal at little or no expense to the underlying relevant signal. In some examples, the ultrasound data may be filtered after interpolation to the pre-set number of frames.

In some embodiments, the pressure processor 170 may be implemented by one or more processors and/or application specific integrated circuits. In some embodiments, the pressure processor 170 may include any one or more machine learning models, artificial intelligence algorithms, and/or neural networks. In some examples, pressure processor 170 may include a partial least squares (PLS) model, deep neural network (DNN), a convolutional neural network (CNN), a recurrent neural network (RNN), support vector machine (SVM), an autoencoder neural network, or the like, to estimate volume and/or determine the pressure. The model and/or neural network may be implemented in hardware (e.g., neurons are represented by physical components) and/or software (e.g., neurons and pathways implemented in a software application) components. The model and/or neural network implemented according to the present disclosure may use a variety of topologies and learning algorithms for training the model and/or neural network to produce the desired output. For example, a software-based neural network may be implemented using a processor (e.g., single or multi-core CPU, a single GPU or GPU cluster, or multiple processors arranged for parallel-processing) configured to execute instructions, which may be stored in computer readable medium, and which when executed cause the processor to perform a trained algorithm for estimating volume and/or determining the pressure. In some embodiments, the pressure processor 170 may implement a model and/or neural network in combination with other image processing methods (e.g., segmentation, histogram analysis).

In various embodiments, the model(s) and/or neural network(s) may be trained using any of a variety of currently known or later developed learning techniques to obtain a model and/or neural network (e.g., a trained algorithm, transfer function, or hardware-based system of nodes) that is configured to analyze input data in the form of ultrasound images, measurements, and/or statistics. In some embodiments, the model and/or neural network may be statically trained. That is, the model and/or neural network may be trained with a data set and deployed on the pressure processor 170. In some embodiments, the model and/or neural network may be dynamically trained. In these embodiments, the model and/or neural network may be trained with an initial data set and deployed on the pressure processor 170. However, the model and/or neural network may continue to train and be modified based on ultrasound images acquired by the pressure processor 170 after deployment of the model and/or neural network on the pressure processor 170.

In some examples, the ultrasound data may be provided to a model of the pressure processor 170. The model may determine a value of the pressure based on an analysis of the ultrasound data. The ultrasound data may have been preprocessed as described above prior to being provided to the model. The model may include a correlation algorithm that correlates the ultrasound data to a value of pressure. The correlation algorithm may have been trained to generate a transfer function with one or more regression coefficients. In some examples, the transfer function may be a matrix of one or more dimensions. The transfer function may be applied to the ultrasound data to output the value of the pressure. In some examples, the correlation algorithm includes a PLS model. The PLS technique may allow for correlation between a set of multiple inputs (e.g., volume, strain measurements) and a single output (e.g., LVEDP), even when the inputs are linearly correlated with one another. In some examples, the model may include a neural network, such as a Long Short-Term Memory (LSTM) network, in addition to or instead of the PLS model.

Alternatively or in addition to a value of pressure a classifier may be output by the pressure processor 170. For example, a binary classifier (e.g., normal/high), or a multi-level classifier (e.g., normal, mild, moderate, severe or normal, high, low). The level of the classifier may be based, at least in part, on whether a value of the pressure is above, equal to, or below one or more threshold values. For example, in the case of a binary classifier, the pressure processor 170 may output "normal" as a level of the classifier if the pressure is below a threshold value and output "high" if the pressure is equal to or above the threshold value. Threshold values for pressure may be based, at least in part, on characteristics of the subject (e.g., sex, age, weight) in some examples.

Optionally, in some examples, the pressure processor 170 may output additional data such as a confidence level associated with the value of the pressure. In some examples, the confidence level may be used to provide additional classifiers. For example, if there is insufficient data and/or the data is too noisy for the pressure processor 170 to determine a value of the pressure with confidence above a threshold value (e.g., 50%, 60%, 80%, 90%, 95%), the classifier returned may be indeterminate (e.g., unknown, invalid). Furthermore, although strain and volume are provided as examples, other ultrasound data may also be provided to the pressure processor 170 for analysis (e.g., blood flow velocity estimates from the heart over a cardiac cycle) to determine the pressure. Additionally, in some examples, non-ultrasound data may also be provided to the pressure processor 170 for analysis (e.g., electrocardiography signals) to determine the pressure.

Outputs from the pressure processor 170, scan converter 130, the multiplanar reformatter 132, and/or the volume renderer 134 may be coupled to an image processor 136 for further enhancement, buffering and temporary storage before being displayed on an image display 138. In some examples, the value of the pressure and/or a classifier associated with the pressure may be shown on the display 138 as text and/or a color. For example, the classifier may be displayed as a circle or other symbol and the color of the symbol may indicate a level of the classifier (e.g., normal=green, mild=yellow, moderate=orange, high=red, unknown/invalid=gray). In some examples, the value of pressure and/or classifier may be shown on the display 138 simultaneously with one or more images (e.g., B-mode image, strain map). A graphics processor 140 may generate graphic overlays for display with the images. These graphic overlays can contain, e.g., standard identifying information such as patient name, date and time of the image, imaging parameters, and the like. For these purposes the graphics processor may be configured to receive input from the user interface 124, such as a typed patient name or other annotations. The user interface 124 can also be coupled to the multiplanar reformatter 132 for selection and control of a display of multiple multiplanar reformatted (MPR) images.

The system 100 may include local memory 142. Local memory 142 may be implemented as any suitable non-transitory computer readable medium (e.g., flash drive, disk drive). Local memory 142 may store data generated by the system 100 including ultrasound images, strain measurements, volume measurements, executable instructions, imaging parameters, training data sets, and/or any other information necessary for the operation of the system 100.

As mentioned previously system 100 includes user interface 124. User interface 124 may include display 138 and control panel 152. The display 138 may include a display device implemented using a variety of known display technologies, such as LCD, LED, OLED, or plasma display technology. In some embodiments, display 138 may comprise multiple displays. The control panel 152 may be configured to receive user inputs (e.g., pre-set number of frames, filter window length, imaging mode). The control panel 152 may include one or more hard controls (e.g., buttons, knobs, dials, encoders, mouse, trackball or others). In some embodiments, the control panel 152 may additionally or alternatively include soft controls (e.g., GUI control elements or simply, GUI controls) provided on a touch sensitive display. In some embodiments, display 138 may be a touch sensitive display that includes one or more soft controls of the control panel 152.

In some embodiments, various components shown in Fig. 1 may be combined. For instance, image processor 136 and graphics processor 140 may be implemented as a single processor. In some embodiments, various components shown in Fig. 1 may be implemented as separate components. For example, signal processor 126 may be implemented as separate signal processors for each imaging mode (e.g., B-mode, Doppler, SWE). In some embodiments, one or more of the various processors shown in Fig. 1 may be implemented by general purpose processors and/or microprocessors configured to perform the specified tasks. In some embodiments, one or more of the various processors may be implemented as application specific circuits. In some embodiments, one or more of the various processors (e.g., image processor 136) may be implemented with one or more graphical processing units (GPU).

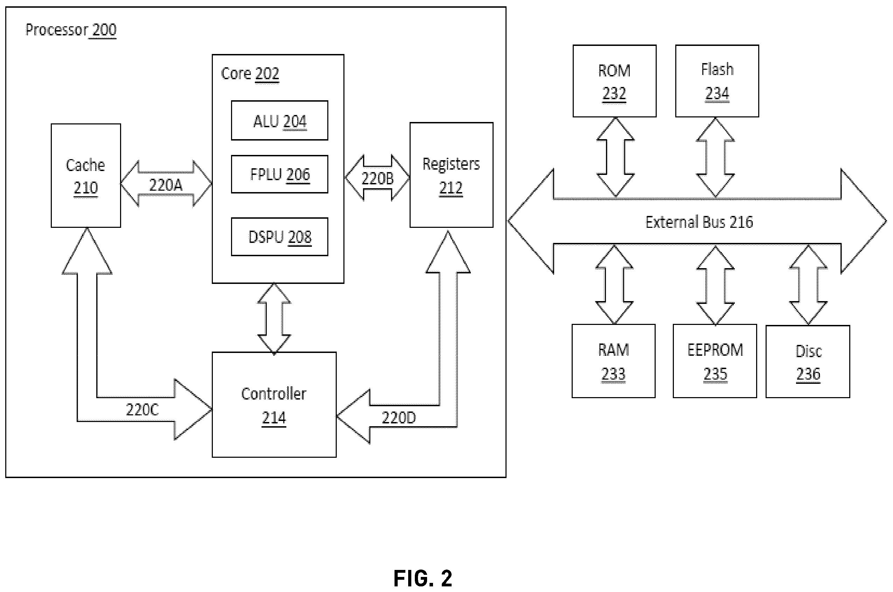

Fig. 2 is a block diagram illustrating an example processor 200 according to principles of the present disclosure. Processor 200 may be used to implement one or more processors and/or controllers described herein, for example, image processor 136 shown in Fig. 1 and/or any other processor or controller shown in Fig. 1. Processor 200 may be any suitable processor type including, but not limited to, a microprocessor, a microcontroller, a digital signal processor (DSP), a field programmable array (FPGA) where the FPGA has been programmed to form a processor, a graphical processing unit (GPU), an application specific circuit (ASIC) where the ASIC has been designed to form a processor, or a combination thereof.

The processor 200 may include one or more cores 202. The core 202 may include one or more arithmetic logic units (ALU) 204. In some embodiments, the core 202 may include a floating point logic unit (FPLU) 206 and/or a digital signal processing unit (DSPU) 208 in addition to or instead of the ALU 204.

The processor 200 may include one or more registers 212 communicatively coupled to the core 202. The registers 212 may be implemented using dedicated logic gate circuits (e.g., flip-flops) and/or any memory technology. In some embodiments the registers 212 may be implemented using static memory. The register may provide data, instructions and addresses to the core 202.

In some embodiments, processor 200 may include one or more levels of cache memory 210 communicatively coupled to the core 202. The cache memory 210 may provide computer-readable instructions to the core 202 for execution. The cache memory 210 may provide data for processing by the core 202. In some embodiments, the computer-readable instructions may have been provided to the cache memory 210 by a local memory, for example, local memory attached to the external bus 216. The cache memory 210 may be implemented with any suitable cache memory type, for example, metal-oxide semiconductor (MOS) memory such as static random access memory (SRAM), dynamic random access memory (DRAM), and/or any other suitable memory technology.

The processor 200 may include a controller 214, which may control input to the processor 200 from other processors and/or components included in a system (e.g., control panel 152 and scan converter 130 shown in Fig. 1) and/or outputs from the processor 200 to other processors and/or components included in the system (e.g., display 138 and volume renderer 134 shown in Fig. 1). Controller 214 may control the data paths in the ALU 204, FPLU 206 and/or DSPU 208. Controller 214 may be implemented as one or more state machines, data paths and/or dedicated control logic. The gates of controller 214 may be implemented as standalone gates, FPGA, ASIC or any other suitable technology.

The registers 212 and the cache memory 210 may communicate with controller 214 and core 202 via internal connections 220A, 220B, 220C and 220D. Internal connections may implemented as a bus, multiplexor, crossbar switch, and/or any other suitable connection technology.

Inputs and outputs for the processor 200 may be provided via a bus 216, which may include one or more conductive lines. The bus 216 may be communicatively coupled to one or more components of processor 200, for example the controller 214, cache memory 210, and/or register 212. The bus 216 may be coupled to one or more components of the system, such as display 138 and control panel 152 mentioned previously.

The bus 216 may be coupled to one or more external memories. The external memories may include Read Only Memory (ROM) 232. ROM 232 may be a masked ROM, Electronically Programmable Read Only Memory (EPROM) or any other suitable technology. The external memory may include Random Access Memory (RAM) 233. RAM 233 may be a static RAM, battery backed up static RAM, Dynamic RAM (DRAM) or any other suitable technology. The external memory may include Electrically Erasable Programmable Read Only Memory (EEPROM) 235. The external memory may include Flash memory 234. The external memory may include a magnetic storage device such as disc 236. In some embodiments, the external memories may be included in a system, such as ultrasound imaging system 100 shown in Fig. 1, for example local memory 142.

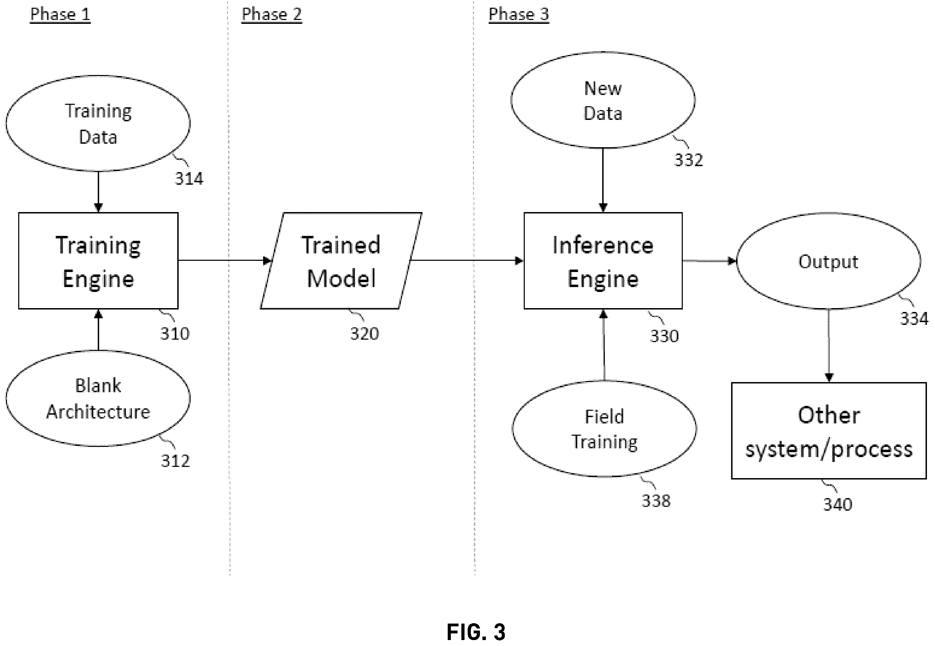

Fig. 3 shows a block diagram of a process for training and deployment of a model such as a correlation algorithm and/or a neural network (e.g., PLS, LSTM) in accordance with the principles of the present disclosure. The process shown in Fig. 3 may be used to train a model included in the pressure processor 170. The left hand side of Fig. 3, phase 1, illustrates the training of a model. To train the model, training sets which include multiple instances of input arrays and output classifications may be presented to the training algorithm(s) of the model(s) (e.g., AlexNet training algorithm, as described by Krizhevsky, A., Sutskever, I. and Hinton, *G. E. "ImageNet Classification with Deep Convolutional Neural Networks,*" NIPS 2012 or its descendants). Training may involve the selection of a starting algorithm and/or network architecture 312 and the preparation of training data 314. The starting architecture 312 may be a blank architecture (e.g., an architecture with defined layers and arrangement of nodes but without any previously trained weights, a defined algorithm with or without a set number of regression coefficients) or a partially trained model, such as the inception networks, which may then be further tailored for analysis of ultrasound data. The starting architecture 312 (e.g., blank weights) and training data 314 are provided to a training engine 310 for training the model. Upon sufficient number of iterations (e.g., when the model performs consistently within an acceptable error), the model 320 is said to be trained and ready for deployment, which is illustrated in the middle of Fig. 3, phase 2. The right hand side of Fig. 3, or phase 3, the trained model 320 is applied (via inference engine 330) for analysis of new data 332, which is data that has not been presented to the model during the initial training (in phase 1). For example, the new data 332 may include unknown data such as live ultrasound images acquired during a scan of a patient (e.g., cardiac images during an echocardiography exam). The trained model 320 implemented via engine 330 is used to analyze the unknown data in accordance with the training of the model 320 to provide an output 334 (e.g., a border of the LA, a value of pressure.). The output 334 may then be used by the system for subsequent processes 340 (e.g., determining a volume of the LA from the border, outputting text of the pressure on a display).

In the embodiments where the trained model 320 is used to implement a model of the pressure processor 170, the starting architecture may be that of a convolutional neural network, or a deep convolutional neural network in some examples, which may be trained to generate the border of the LA, LV, and/or other heart chamber. The training data 314 may include multiple (hundreds, often thousands or even more) annotated/labeled images, also referred to as training images. It will be understood that the training image need not include a full image produced by an imaging system (e.g., representative of the full field of view of an ultrasound probe) but may include patches or portions of images, for example, those portions that include the LA.

In embodiments where the trained model 320 is used to implement a model of the pressure processor 170, the starting architecture may be that of a PLS model and/or a LSTM network in some examples, which may be trained to generate a value and/or a classifier for the pressure. The training data 314 may include multiple ultrasound data sets (e.g., strain curves, volumes, etc.) annotated/labeled with the pressure measured by a catheter or other means. In some examples, the training data 314 may be preprocessed as described with reference to Fig. 1 prior to being provided to the training engine 310. In some examples, the ultrasound data sets may be annotated/labeled with a classifier (e.g., high, low). In some examples, the ultrasound data sets may be annotated/labeled with both the classifier and the value of the pressure.

In some examples, training may include providing a training data set, a validation data set, and a test data set. The training data set may be used for algorithm building (e.g., finding the weights of the coefficients in the PLS and/or LSTM network). The validation data set may be used to optimize the model after training to avoid overfitting to the training data set. For example, when a loss function of the validation data set increases for three consecutive training epochs, training may be stopped. Finally, the trained model may be tested on the test data set. In some applications, additional data may be used for training the LSTM network compared to the PLS model.

In various embodiments, the trained model(s) may be implemented, at least in part, in a computer-readable medium comprising executable instructions executed by a processor, e.g., pressure processor 170.

Fig. 4 illustrates an example of a strain curve in accordance with the principles of the present disclosure.

In some examples, the longitudinal strain curve 400 is generated using the strain processor 166 of ultrasound imaging system 100 based on ultrasound data.

The example longitudinal strain curve 400 for the LA (e.g., LA strain curve is shown in may be used as an input, in combination with other parameters, to the correlation algorithm to detect/predict various heart related and non-heart related conditions and diagnoses of the patient. The example strain curve 400 reflects the percentage strain of the LA tissue over time in milliseconds. Note that the LA strain curve is generated based on an ultrasound performed on the patient. Alternatively, or in addition, an LV strain curve, or other strain measurements may be generated and used as an input to the multi-parametric correlation algorithm to detect/predict the various conditions and/or diagnoses in a patient.

Fig. 5 illustrates an example of a longitudinal global strain curve 506 in accordance with the principles of the present disclosure.

In some examples, the longitudinal PSS strain curves 502, the longitudinal ESS strain curves 504 and the longitudinal global strain curve 506 are generated using the strain processor 166 of ultrasound imaging system 100 based on ultrasound data.

The example longitudinal global strain curve 506 may be used as an input (e.g., or an additional input) to the multi-parametric correlation algorithm to detect various heart related and non-heart related conditions and/or diagnoses of the patient. The longitudinal global strain curve 506 is an average of a plurality of longitudinal PSS strain curves 502 or an average of a plurality of longitudinal ESS strain curves 504 that are generated based on ultrasound data. The longitudinal PSS strain curves 502 and the longitudinal ESS strain curves 504 reflect the percentage strain of tissue (e.g., LA or LV) over time in milliseconds at the PSS and at the ESS respectively. The longitudinal global strain curve 506 reflects the average percentage strain of tissue (e.g., LA or LV) over time in milliseconds. In some instances, the longitudinal global strain curve 506 may be an average of the plurality of longitudinal PSS strain curves 502 and the plurality of longitudinal ESS strain curves 504. In various embodiments, the plurality of longitudinal PSS strain curves 502 and/or the plurality of longitudinal ESS strain curves 504 may be used as inputs to the correlation algorithm or may be used to train the correlation algorithm.

The strain curves shown in Figs. 4 and 5 are provided merely as examples, and other strain measurements may be used to train the one or more models for determining cardiac pressure. Further, other parameters may be used to determine cardiac pressure such as volume, ejection fraction, jet velocity, and the like.

Fig. 6 is an illustration of a neural network that may be used to analyze data in accordance with examples of the present disclosure. In some examples, the neural network 600 may be implemented by one or more processors (e.g., image processor 136, graphics processor 140, pressure processor 170) of an ultrasound imaging system, such as ultrasound imaging system 100. In some examples, neural network 600 may be a convolutional network with single and/or multidimensional layers. The neural network 600 may include one or more input nodes 602. In some examples, the input nodes 602 may be organized in a layer of the neural network 600. The input nodes 602 may be coupled to one or more layers 608 of hidden units 606 by weights 604. In some examples, the hidden units 606 may perform operations on one or more inputs from the input nodes 602 based, at least in part, with the associated weights 604. In some examples, the hidden units 606 may be coupled to one or more layers 614 of hidden units 612 by weights 610. The hidden units 612 may perform operations on one or more outputs from the hidden units 606 based, at least in part, on the weights 610. The outputs of the hidden units 612 may be provided to an output node 616 to provide an output (e.g., inference, determination, prediction) of the neural network 600. Although one output node 616 is shown in Fig. 6, in some examples, the neural network may have multiple output nodes 616. In some examples, the output may be accompanied by a confidence level. The confidence level may be a value from, and including, 0 to 1, where a confidence level 0 indicates the neural network 600 has no confidence that the output is correct and a confidence level of 1 indicates the neural network 600 is 100% confident that the output is correct.

In some examples, inputs to the neural network 600 provided at the one or more input nodes 602 may include strain maps, strain curves, tissue stiffness values, echo parameters, and/or previously obtained patient information. In some examples, outputs provided at the output node 616 may include a detection/determination/prediction of a condition/diagnosis (e.g., LVEDP, cardiotoxicity, etc.) and/or a level of the condition/diagnosis (e.g., normal, low, or high cardiac pressure.

Fig. 7 is an illustration of a cell of a long short term memory (LSTM) model that may be used to analyze data in accordance with examples of the present disclosure. In some examples, the LSTM model may be implemented by one or more processors (e.g., image processor 136, graphics processor 140, pressure processor 170) of an ultrasound imaging system such as ultrasound imaging system 100. A LSTM model is a type of recurrent neural network that is capable learning long-term dependencies. An LSTM model typically includes multiple cells coupled together. The number of cells may be based, at least in part, on a length of a sequence to be analyzed by the LSTM. For simplicity, only a single cell 700 is shown in Fig. 7.

The variable C, running across the top of cell 700 is the state of the cell. The state of the previous LSTM cell Cₜ₋₁ may be provided to cell 700 as an input. Data can be selectively added or removed from the state of the cell by cell 700. The addition or removal of data is controlled by three "gates," each of which includes a separate neural network layer. The modified or unmodified state of cell 700 may be provided by cell 700 to the next LSTM cell as Cₜ.

The variable h, running across the bottom of the cell 700 is the hidden state vector of the LSTM model. The hidden state vector of the previous cell hₜ₋₁ may be provided to cell 700 as an input. The hidden state vector hₜ₋₁ may be modified by a current input xₜ to the LSTM model provided to cell 700. The hidden state vector may also be modified based on the state of the cell 700 Cₜ. The modified hidden state vector of cell 700 may be provided as an output hₜ. The output hₜ may be provided to the next LSTM cell as a hidden state vector and/or provided as an output of the LSTM model.

Turning now to the inner workings of cell 700, a first gate (e.g., the forget gate) for controlling a state of the cell C includes a first layer 702. In some examples, this first layer is a sigmoid layer. The sigmoid layer may receive a concatenation of the hidden state vector hₜ₋₁ and the current input xₜ. The first layer 702 provides an output fₜ, which includes weights that indicate which data from the previous cell state should be "forgotten" and which data from the previous cell state should be "remembered" by cell 700. The previous cell state Cₜ₋₁ is multiplied by fₜ at point operation 704 to remove any data that was determined to be forgotten by the first layer 702.

A second gate (e.g., the input gate) includes a second layer 706 and a third layer 710. Both the second layer 706 and the third layer 710 receive the concatenation of the hidden state vector hₜ₋₁ and the current input xₜ. In some examples, the second layer 706 is a sigmoid function. The second layer 706 provides an output it which includes weights that indicate what data needs to be added to the cell state C. The third layer 710 may include a tanh function in some examples. The third layer 710 may generate a vector Ĉₜ that includes all possible data that can be added to the cell state from hₜ₋₁ and xₜ. The weights it and vector Cₜ are multiplied together by point operation 708. The point operation 708 generates a vector that includes the data to be added to the cell state C. The data is added to the cell state C to get the current cell state Cₜ at point operation 712.

A third gate (e.g., the output gate) includes a fourth layer 714. In some examples, the fourth layer 714 is a sigmoid function. The fourth layer 714 receives the concatenation of the hidden state vector hₜ₋₁ and the current input xₜ and provides an output oₜ which includes weights that indicate what data of the cell state Cₜ should be provided as the hidden state vector hₜ of cell 700. The data of the cell state Cₜ is turned into a vector by a tanh function at point operation 716 and is then multiplied by oₜ by point operation 718 to generate the hidden state vector/output vector hₜ. In some examples, the output vector hₜ may be accompanied by a confidence value, similar to the output of a convolutional neural network, such as the one described in reference to Fig. 6.

As pictured in Fig. 7, cell 700 is a "middle" cell. That is, the cell 700 receives inputs Cₜ₋₁ and hₜ₋₁ from a previous cell in the LSTM model and provides outputs Cₜ and hₜ to a next cell in the LSTM. If cell 700 were a first cell in the LSTM, it would only receive input xₜ. If cell 700 were a last cell in the LSTM, the outputs hₜ and Cₜ would not be provided to another cell.

In some examples where a processor of an ultrasound imaging system (e.g., image processor 136, graphics processor 140, pressure processor 170) implements an LSTM model, the current input xₜ may include data related strain maps, strain curves, tissue stiffness values, echo parameters, and/or previously obtained patient information. The hidden state vector hₜ₋₁ may include data related to a previous detection/determination/prediction such as a condition/diagnosis level, for example, a cardiac pressure level such as LVEDP. The cell state Cₜ₋₁ may include data related to previous strain maps, strain curves, and/or echo parameters. In some examples, output(s) hₜ of the LSTM model may be used by the processor and/or another processor of the ultrasound imaging system to apply an indication of the detected/determined/predicted condition/diagnosis to an ultrasound image.

Fig. 8 illustrates an example SVM model in accordance with the principles of the present disclosure.. In some examples, the SVM model may be implemented by one or more processors (e.g., image processor 136, graphics processor 140, pressure processor 170) of an ultrasound imaging system such as ultrasound imaging system 100. A SVM model is a type of machine learning model that is capable of taking in multiple different parameters (e.g., features) as an input. Accordingly, SVM models may be suitable for analyzing and predicting data where multiple different parameters (e.g., features) are used as inputs/used to train the model.

For example, as illustrated in Fig. 8, various data points are plotted according to a first feature 802 (e.g., Y-axis) and a second feature 804 (e.g., X-axis). A vector may be generated according to the most extreme data point (e.g., furthest from the data point grouping) for each grouping of data points. These vectors are referred to as support vectors 810. A hyperplane 812 is generated based on the support vectors 810 (e.g., an average of the support vectors 810). The hyperplane 812 may be understood as a design boundary which separates data points and classifies which grouping (e.g., the first group 806 of the second group 808) the data points belong in. The data points below the hyperplane 812 are classified into a first group 806 and the data points above the hyperplane 812 are classified into a second group 808.

A margin 814 is determined as the margin between the two support vectors 810. If the margin 814 is small, the support vectors 810 may not be accurate or the dataset may not include different groupings. If the margin 814 is large the support vectors 810 may be accurate and the dataset may include groupings that the data points may be classified into.

While the example illustrated in Fig. 8 is a two dimensional, an SVM model may be used for any dimension of parameters (e.g., features). For example, data points may have multiple features (e.g., two, three, four,...,N) and the SVM may generate the hyperplane 812 for the dataset. However, instead of the hyperplane 812 being a vector/line as illustrated in Fig. 8, it may be a plane for three dimensional datasets and so on for higher dimensional datasets.

In various embodiments, the multi-parametric correlation algorithm may be understood as an SVM model. For example, the SVM model may be used to detect/predict various conditions (e.g., cardiac pressure) and/or diagnoses based on various measurements (e.g., strain measurements, LA index, LV index, echo parameters etc.)inputted into the SVM model. The SVM model may use one or more of the LV strain data, the LA strain data, the RV strain data, the LA index, the LV index, echo parameters, or previously known patient data as an input dataset and generate a hyperplane 812 based on multiple features of the dataset. In some cases, the generated hyperplane 812 may be understood as a level of a condition or diagnosis of the patient (e.g., heart related or non-heart related). For example, the generated hyperplane 812 may be understood as a LVEDP level, a level of cardiotoxicity, and/or other heart condition.

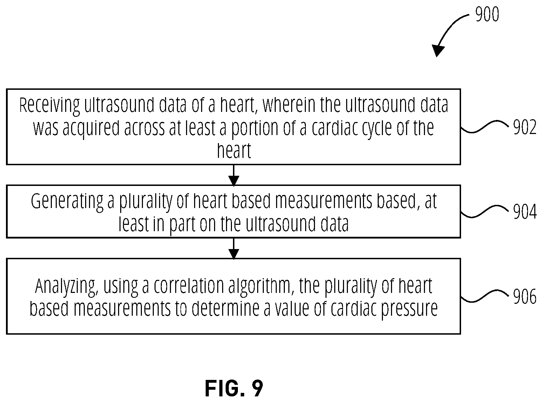

Fig. 9 illustrates a method 900, according to embodiments herein. The illustrated method 900 includes receiving 902 ultrasound data of a heart, wherein the ultrasound data was acquired across at least a portion of a cardiac cycle of the heart. The method 900 further includes generating 904 a plurality of heart based measurements based, at least in part on the ultrasound data. The method 900 further includes analyzing 906, using a correlation algorithm, the plurality of heart based measurements to determine a value of cardiac pressure.

In some embodiments of the method 900, the correlation algorithm comprises an SVM model trained on one or more of an LA index, an LV index, LA volume parameters, echo parameters, LV strain data, LA strain data, RA strain data, RV strain data, ultrasound Doppler data, ultrasound pulsed wave Doppler data, ultrasound continuous wave Doppler data, ultrasound two dimensional color data, and ultrasound three dimensional color data. For example, the pressure processor 170 uses a SVM model trained on the strain measurements generated by the strain processor 166.

In some embodiments of the method 900, the plurality of heart based measurements comprise at least two of an LA index, an LV index, LA volume parameters, echo parameters, LV strain data, LA strain data, RA strain data, RV strain data, ultrasound Doppler data, ultrasound pulsed wave Doppler data, ultrasound continuous wave Doppler data, ultrasound two dimensional color data, and ultrasound three dimensional color data. For example, the strain processor 166 generates the strain measurements such as the LV strain data, the LA strain data, the RV strain data, the LA index, or the LV index.

In some embodiments, the method 900 further comprises weighting one or more of the plurality of heart based measurements, and analyzing, using the correlation algorithm, the weighted one or more of the plurality of heart based measurements to determine the value of cardiac pressure. For example, the pressure processor 170 may weight the plurality of heart based measurements.

In some embodiments, the method 900 further comprises filtering the ultrasound data with a digital filter, and wherein the digital filter includes a Savitsky-Golay filter with a cubic polyfit. For example, the signal processor 126 may filter the ultrasound data with a digital filter.

In some embodiments, the method 900 further comprises interpolating the ultrasound data to a pre-set number of frames across the at least the portion of the cardiac cycle. For example, the signal processor 126 may interpolate the ultrasound data.

In some embodiments, the method 900 further comprises generating a classifier associated with the value of the cardiac pressure. For example, the pressure processor 170 may generate a classifier associated with the value of the cardiac pressure.

In various embodiments, the multi-parametric correlation algorithm may detect/predict/determine conditions and/or diagnoses based on a plurality of input measurements (e.g., determined from the ultrasound data), thus producing a more accurate and more reliable detection/prediction/determination that is not achievable by current methods. Note that the more data and diversity of data that the multi-parametric correlation algorithm is provided, the more accurate the determination is, as the model is provided more contextual data of the patient.

Although the examples described herein discuss processing of ultrasound image data, it is understood that the principles of the present disclosure are not limited to ultrasound and may be applied to image data from other modalities such as magnetic resonance imaging and computed tomography.

In various embodiments where components, systems and/or methods are implemented using a programmable device, such as a computer-based system or programmable logic, it should be appreciated that the above-described systems and methods can be implemented using any of various known or later developed programming languages, such as "C", "C++", "C#", "Java", "Python", and the like. Accordingly, various storage media, such as magnetic computer disks, optical disks, electronic memories and the like, can be prepared that can contain information that can direct a device, such as a computer, to implement the above-described systems and/or methods. Once an appropriate device has access to the information and programs contained on the storage media, the storage media can provide the information and programs to the device, thus enabling the device to perform functions of the systems and/or methods described herein. For example, if a computer disk containing appropriate materials, such as a source file, an object file, an executable file or the like, were provided to a computer, the computer could receive the information, appropriately configure itself and perform the functions of the various systems and methods outlined in the diagrams and flowcharts above to implement the various functions. That is, the computer could receive various portions of information from the disk relating to different elements of the above-described systems and/or methods, implement the individual systems and/or methods and coordinate the functions of the individual systems and/or methods described above.

In view of this disclosure it is noted that the various methods and devices described herein can be implemented in hardware, software and firmware. Further, the various methods and parameters are included by way of example only and not in any limiting sense. In view of this disclosure, those of ordinary skill in the art can implement the present teachings in determining their own techniques and needed equipment to affect these techniques, while remaining within the scope of the invention. The functionality of one or more of the processors described herein may be incorporated into a fewer number or a single processing unit (e.g., a CPU) and may be implemented using application specific integrated circuits (ASICs) or general purpose processing circuits which are programmed responsive to executable instruction to perform the functions described herein.

Although the present system may have been described with particular reference to an ultrasound imaging system, it is also envisioned that the present system can be extended to other medical imaging systems where one or more images are obtained in a systematic manner. Accordingly, the present system may be used to obtain and/or record image information related to, but not limited to renal, testicular, breast, ovarian, uterine, thyroid, hepatic, lung, musculoskeletal, splenic, cardiac, arterial and vascular systems, as well as other imaging applications related to ultrasound-guided interventions. Further, the present system may also include one or more programs which may be used with conventional imaging systems so that they may provide features and advantages of the present system. Certain additional advantages and features of this disclosure may be apparent to those skilled in the art upon studying the disclosure, or may be experienced by persons employing the novel system and method of the present disclosure. Another advantage of the present systems and method may be that conventional medical image systems can be easily upgraded to incorporate the features and advantages of the present systems, devices, and methods.

Of course, it is to be appreciated that any one of the examples, embodiments or processes described herein may be combined with one or more other examples, embodiments and/or processes or be separated and/or performed amongst separate devices or device portions in accordance with the present systems, devices and methods.

Finally, the above-discussion is intended to be merely illustrative of the present system and should not be construed as limiting the appended claims to any particular embodiment or group of embodiments. Thus, while the present system has been described in particular detail with reference to exemplary embodiments, it should also be appreciated that numerous modifications and alternative embodiments may be devised by those having ordinary skill in the art without departing from the broader and intended spirit and scope of the present system as set forth in the claims that follow. Accordingly, the specification and drawings are to be regarded in an illustrative manner and are not intended to limit the scope of the appended claims.

## Claims

1. An ultrasound imaging system comprising:
a processor configured to:
receive ultrasound data of a heart, wherein the ultrasound data was acquired across at least a portion of a cardiac cycle of the heart;
generate a plurality of heart based measurements based, at least in part on the ultrasound data; and
analyze, using a correlation algorithm, the plurality of heart based measurements to determine a value of cardiac pressure,
wherein the correlation algorithm comprises a support vector machine, SVM, model; and wherein the plurality of heart based measurements comprise at least two of: a left atrial, LA, index; a left ventricular, LV, index; LA volume parameters; echo parameters; LV strain data; LA strain data; right atrial, RA, strain data; right ventricular, RV, strain data; ultrasound Doppler data; ultrasound pulsed wave Doppler data; ultrasound continuous wave Doppler data; ultrasound two dimensional color data; and ultrasound three dimensional color data.

2. The ultrasound imaging system of claim 1, wherein the SVM model is trained on one or more of an LA index, an LV index, LA volume parameters, echo parameters, LV strain data, LA strain data, RA strain data, RV strain data, ultrasound Doppler data, ultrasound pulsed wave Doppler data, ultrasound continuous wave Doppler data, ultrasound two dimensional color data, and ultrasound three dimensional color data.

3. The ultrasound imaging system of claim 1 or 2, wherein the processor is further configured to:
weight one or more of the plurality of heart based measurements; and
analyze, using the correlation algorithm, the weighted one or more of the plurality of heart based measurements to determine the value of cardiac pressure.

4. The ultrasound imaging system of any of claims 1-3, wherein the plurality of heart based measurements comprises one or more global LV strain curves, and wherein the one or more global LV strain curves comprise an average of a plurality of LV strain curves.

5. The ultrasound imaging system of any of claims 1-4, wherein the processor is further configured to:
analyze a sequence of two-dimensional ultrasound images with a machine learning model to determine a border of a chamber of the heart in individual ones of the two-dimensional ultrasound images; and
calculate volumes of the chamber, based, at least in part, on the borders of the individual ones of the two-dimensional ultrasound images, wherein the volumes of the chamber are included in the ultrasound data.

6. The ultrasound imaging system of any of claims 1-5, wherein the processor is further configured to:
analyze a sequence of three-dimensional ultrasound images with a machine learning model to determine a border of a chamber of the heart in individual ones of the three-dimensional ultrasound images; and
calculate volumes of the chamber, based, at least in part, on the borders of the individual ones of the three-dimensional ultrasound images, wherein the volumes of the chamber are included in the ultrasound data.

7. A method comprising:
receiving ultrasound data of a heart, wherein the ultrasound data was acquired across at least a portion of a cardiac cycle of the heart;
generating a plurality of heart based measurements based, at least in part on the ultrasound data; and
analyzing, using a correlation algorithm, the plurality of heart based measurements to determine a value of cardiac pressure,
wherein the correlation algorithm comprises a support vector machine, SVM, model; and
wherein the plurality of heart based measurements comprise at least two of: a left atrial, LA, index; a left ventricular, LV, index; LA volume parameters; echo parameters; LV strain data; LA strain data; right atrial, RA, strain data; right ventricular, RV, strain data; ultrasound Doppler data; ultrasound pulsed wave Doppler data; ultrasound continuous wave Doppler data; ultrasound two dimensional color data; and ultrasound three dimensional color data.

8. The method of claim 7, wherein the SVM model is trained on one or more of an LA index, a LV index, LA volume parameters, echo parameters, LV strain data, LA strain data, RA strain data, RV strain data, ultrasound Doppler data, ultrasound pulsed wave Doppler data, ultrasound continuous wave Doppler data, ultrasound two dimensional color data, and ultrasound three dimensional color data.

9. The method of claim 7 or 8, further comprising:
weighting one or more of the plurality of heart based measurements; and
analyzing, using the correlation algorithm, the weighted one or more of the plurality of heart based measurements to determine the value of cardiac pressure.

10. The method of any of claims 7-9, wherein the plurality of heart based measurements comprises one or more global LV strain curves, and wherein the one or more global LV strain curves comprise an average of a plurality of LV strain curves.

11. The method of any of claims 7-10, further comprising filtering the ultrasound data with a digital filter, and wherein the digital filter includes a Savitsky-Golay filter with a cubic polyfit.

12. The method of any of claims 7-11, further comprising interpolating the ultrasound data to a pre-set number of frames across the at least the portion of the cardiac cycle.

13. The method of any of claims 7-12, further comprising generating a classifier associated with the value of the cardiac pressure.

14. The method of any of claims 7-13, further comprising:
analyzing a sequence of ultrasound images with a machine learning model to determine a border of a chamber of the heart in individual ones of the ultrasound images; and
calculating volumes of the chamber, based, at least in part, on the borders of the individual ones of the ultrasound images, wherein the volumes of the chamber are included in the ultrasound data.

15. A non-transitory computer-readable medium carrying instructions which, when executed by a processor, cause the processor to carry out the steps of the method of any of claims 7-14.
